# EUROPEAN PATENT APPLICATION

(11) **EP 3 398 588 A1**
(43) Date of publication of application: **07.11.2018**
(21) Application number: 16881739.3
(22) Date of filing: 26.12.2016
(51) Int. Cl.: A61K 9/20, A61K 9/16, A61K 31/192, A61K 31/196, A61K 47/02, A61K 47/04, A61K 47/12, A61K 47/26, A61K 47/32

(54) **COMPRESSION-MOLDED PREPARATION**

(30) Priority: 28.12.2015 JP 2015255828
(71) Applicant: Nippon Shinyaku Co., Ltd., Kyoto-shi Kyoto 601-8550 (JP)
(72) Inventor: HATTORI, Naoto, Kyoto-shi Kyoto 601-8550 (JP); YASUDA, Akihito, Kyoto-shi Kyoto 601-8550 (JP); SASAKI, Hitoshi, Kyoto-shi Kyoto 601-8550 (JP); ONUKI, Yoichi, Narita-shi Chiba 286-8511 (JP); OSAWA, Ryoichi, Tokyo 103-8481 (JP)
(74) Representative: Adam, Holger
(86) International application number: PCT/JP2016/088787
(87) International publication number: WO 2017/115764

(57) **Abstract**

An object of the present invention is to provide a compression-molded preparation which has an excellent disintegration property and can be easily produced despite the use of granules coated with a polymer coating film having a function such as masking of an unpleasant taste. A compression-molded preparation achieving the above object is characterized by including granules obtained by coating a polymer-coated, granulated substance, in which a granulated substance containing a drug is coated with a polymer coating film, with one kind or two or more kinds of additives selected from the group consisting of a metal stearate, stearic acid, a sucrose fatty acid ester, talc, and silicic acid.

## Description

### Technical Field

The present invention relates to a compression-molded preparation which is obtained by using granules, in which a granulated substance containing a drug and coated with a polymer coating film is further coated with one kind or two or more kinds of additives selected from the group consisting of a metal stearate, stearic acid, a sucrose fatty acid ester, talc, and silicic acid (hereinafter referred to as "specific additive"), and has an excellent disintegration property while having a function such as masking of an unpleasant taste of the drug or control of dissolution of the drug.

### Background Art

As a method for producing a compression-molded preparation, a direct method, in which a mixed powder obtained by adding an additive such as an excipient to a drug, followed by uniformly mixing is directly compression-molded, a granule compression method, in which such a mixed powder is once formed into granules, followed by compression molding, or the like is generally used.

In a compression-molded preparation, various drugs are used, however, for example, a preparation using a drug such as an antipyretic analgesic is sometimes required to have a fast-acting property, and it is necessary to shorten the disintegration time so as to promptly dissolve the drug. Further, recently, research and development of an orally disintegrating tablet as a dosage form which is easily taken even by patients having difficulty in swallowing such as the elderly and children have been conducted. In order to produce such an orally disintegrating tablet by a direct method, a method in which a drug is subjected to surface modification with light anhydrous silicic acid to improve the fluidity of the drug, and then, an excipient is mixed therewith, and the resulting mixture is tableted has been disclosed (PTL 1) . Further, a compression-molded preparation is required to have a hardness such that damage or the like does not occur in the process of production or transport, and a method in which the hardness is enhanced while maintaining a disintegration property by incorporating a lubricant before a compression treatment in a granule compression method has been disclosed (PTL 2).

On the other hand, many of the drugs exhibit astringency, irritancy, a bitter taste, or an unpleasant taste such as an astringent taste or a bitter taste, which makes it difficult to take the drug as it is. Further, for the purpose of effectively expressing the drug efficacy, alleviating adverse effects, etc., a function such as enteric solubility, a sustained release property, a long-lasting property, or gastric juice resistance is imparted to a drug in some cases. In order to impart a function such as masking of an unpleasant taste of a drug or control of dissolution of a drug, granules containing a drug are coated with a gastric-soluble polymer, an enteric-soluble polymer, a water-insoluble polymer, or the like, and then formulated into a preparation.

For example, in order to mask an unpleasant taste of a drug such as fexofenadine hydrochloride, an orally disintegrating tablet obtained by coating the surfaces of granules containing the drug with an aminoalkyl methacrylate copolymer or the like, followed by tableting has been disclosed (PTL 3). Further, in order to mask a flavor and protect from moisture, a coating material using methyl methacrylate/diethylaminoethyl methacrylate or the like is disclosed along with a description of coating of granules or the like with the coating material (PTL 4).

However, the disintegration time of a compression-molded preparation produced using granules coated with a polymer coating film is greatly increased, and therefore, in order to improve the disintegration property, it is required to perform tableting at a low pressure, and so on, which leads to significant constraints on the production and difficulty of stable production.

### Citation List

### Patent Literature

PTL 1: WO 2000/54752
PTL 2: JP 2006-265242A
PTL 3: JP 2013-147470A
PTL 4: JP 2013-509368A

### Summary of Invention

### Technical Problem

An object of the present invention is to provide a compression-molded preparation which has an excellent disintegration property and can be easily produced despite the use of granules coated with a polymer coating film having a function such as masking of an unpleasant taste, and a method for producing the same.

### Solution to Problem

As a result of intensive studies for achieving the above objects, the present inventors found that when compression molding is performed, by mixing a granulated substance containing a drug and coated with a polymer coating film with a specific additive beforehand so as to coat the granulated substance with the specific additive, the disintegration time of a preparation is significantly shortened, and a preparation having a favorable disintegration property is obtained at a wide range of tableting pressure, and thus completed the present invention.

That is, the present invention is a compression-molded preparation including granules obtained by coating a polymer-coated, granulated substance, in which a granulated substance containing a drug is coated with a polymer coating film, with a specific additive.

Further, the present invention is a method for producing a compression-molded preparation characterized in that a polymer-coated, granulated substance is obtained by coating a granulated substance containing a drug with a polymer coating film, and then, granules obtained by coating the polymer-coated, granulated substance with a specific additive are compression-molded.

In addition, the present invention is a granule which is obtained by coating a polymer-coated, granulated substance, in which a granulated substance containing a drug is coated with a polymer coating film, with a specific additive.

### Effects of the Invention

According to the present invention, even if compression molding is performed using granules coated with a polymer coating film, the disintegration time can be greatly shortened. Therefore, a compression-molded preparation which has various functions attributed to the polymer coating film such as masking of an unpleasant taste, and also has an excellent disintegration property can be obtained.

### Brief Description of Drawings

[FIG. 1] FIG. 1 is a graph showing the tablet hardness with respect to each tableting pressure of tablets of Examples 1 to 3 and Comparative Example 1.
[FIG. 2] FIG. 2 is a graph showing the disintegration time with respect to each tableting pressure of the tablets of Examples 1 to 3 and Comparative Example 1.
[FIG. 3] FIG. 3 is a graph showing the tablet hardness with respect to each tableting pressure of tablets of Example 4 and Comparative Example 2.
[FIG. 4] FIG. 4 is a graph showing the disintegration time with respect to each tableting pressure of the tablets of Example 4 and Comparative Example 2.
[FIG. 5] FIG. 5 is a graph showing the tablet hardness with respect to each tableting pressure of tablets of Examples 5 to 7 and Comparative Example 3.
[FIG. 6] FIG. 6 is a graph showing the disintegration time with respect to each tableting pressure of the tablets of Examples 5 to 7 and Comparative Example 3.
[FIG. 7] FIG. 7 is a graph showing the tablet hardness with respect to each tableting pressure of tablets of Example 8 and Comparative Example 4.
[FIG. 8] FIG. 8 is a graph showing the disintegration time with respect to each tableting pressure of the tablets of Example 8 and Comparative Example 4.
[FIG. 9] FIG. 9 is a graph showing the tablet hardness with respect to each tableting pressure of tablets of Example 9 and Comparative Example 5.
[FIG. 10] FIG. 10 is a graph showing the disintegration time with respect to each tableting pressure of the tablets of Example 9 and Comparative Example 5.
[FIG. 11] FIG. 11 is a graph showing the tablet hardness with respect to each tableting pressure of tablets of Example 10 and Comparative Example 6.
[FIG. 12] FIG. 12 is a graph showing the disintegration time with respect to each tableting pressure of the tablets of Example 10 and Comparative Example 6.
[FIG. 13] FIG. 13 is a graph showing the tablet hardness with respect to each tableting pressure of tablets of Example 11 and Comparative Example 7.
[FIG. 14] FIG. 14 is a graph showing the disintegration time with respect to each tableting pressure of the tablets of Example 11 and Comparative Example 7.
[FIG. 15] FIG. 15 is a graph showing the tablet hardness with respect to each tableting pressure of tablets of Example 12 and Comparative Example 8.
[FIG. 16] FIG. 16 is a graph showing the disintegration time with respect to each tableting pressure of the tablets of Example 12 and Comparative Example 8.
[FIG. 17] FIG. 17 is a graph showing the tablet hardness with respect to each tableting pressure of tablets of Example 13 and Comparative Example 9.
[FIG. 18] FIG. 18 is a graph showing the disintegration time with respect to each tableting pressure of the tablets of Example 13 and Comparative Example 9.

### Description of Embodiments

A drug to be used in the present invention is not particularly limited, and there can be exemplified a drug which has an unpleasant taste and needs to be masked, a drug to which enteric solubility or gastric solubility needs to be imparted, a drug to which acid resistance needs to be imparted, a drug to which a sustained release property needs to be imparted, and the like. Specific examples of such a drug include acetaminophen, anhydrous caffeine, clemastine fumarate, promethazine hydrochloride, mequitazine, diphenhydramine hydrochloride, dl-chlorpheniramine maleate, phenylephrine hydrochloride, ibuprofen sodium, loxoprofen sodium, diclofenac potassium, diclofenac sodium, naproxen sodium, methylephedrine hydrochloride, ephedrine hydrochloride, dextromethorphan, noscapine hydrochloride, methylephedrine hydrochloride, bromhexine hydrochloride, and salicylamide, or hydrates thereof, and among these, one kind or two or more kinds can be used.

Among these, ibuprofen sodium, loxoprofen sodium, diclofenac potassium, diclofenac sodium, naproxen sodium, or a hydrate thereof is preferred, and loxoprofen sodium is particularly preferred.

In the present invention, the content of the drug is not particularly limited and can be appropriately determined according to the tolerated dose in oral administration of each drug, or the like. For example, in the case where the drug is loxoprofen sodium, the content of the drug in the preparation is preferably from 1 to 98 mass% (hereinafter simply referred to as "%"), more preferably from 2 to 90%.

In the present invention, the polymer to be used for the purpose of masking an unpleasant taste such as a bitter taste or irritancy of the drug or controlling the dissolution of the drug is not particularly limited, and there can be exemplified a water-soluble polymer such as a cellulosic or methacrylic polymer, a water-insoluble polymer, a gastric-soluble polymer, an enteric-soluble polymer, and the like, and among these, one kind or two or more kinds can be used.

Examples of the water-soluble polymer include methyl cellulose, hypromellose, hydroxypropyl cellulose, polyethylene glycol, polyvinyl alcohol (a partially saponified product), and polyvinylpyrrolidone.

Examples of the water-insoluble polymer include water-insoluble methacrylic polymer compounds such as an ethyl acrylate-methyl methacrylate copolymer (for example, an ethyl acrylate-methyl methacrylate copolymer dispersion liquid (Eudragit NE30D)) and an ethyl acrylate-methyl methacrylate-ethyl methacrylate trimethylammonium chloride copolymer (for example, aminoalkyl methacrylate copolymer RS (Eudragit RS100, Eudragit RSPO, Eudragit RL, or Eudragit RLPO), and an aminoalkyl methacrylate copolymer RS aqueous dispersion liquid (Eudragit RS30D or Eudragit RL30D)); and water-insoluble cellulosic polymer compounds such as ethyl cellulose (for example, Ethocel or Aquacoat) .

Examples of the gastric-soluble polymer include polyvinyl acetal-based polymers such as polyvinyl acetal diethylamino acetate (for example, AEA); and gastric-soluble methacrylic polymer compounds such as a methyl methacrylate-butyl methacrylate-dimethylaminoethyl methacrylate copolymer (for example, aminoalkyl methacrylate copolymer E (Eudragit EPO or Eudragit E100)), and a methyl methacrylate-diethylaminoethyl methacrylate copolymer (for example, Kollicoat Smartseal 30D).

Examples of the enteric-soluble polymer include enteric-soluble methacrylic polymer compounds such as a methacrylic acid-ethyl acrylate copolymer (for example, methacrylic acid copolymer LD (Eudragit L30D-55 or Eudragit L100-55)), a methacrylic acid-methyl methacrylate copolymer (for example, methacrylic acid copolymer L (Eudragit L100), methacrylic acid copolymer S (Eudragit S100)), and a methyl acrylate-methyl methacrylate-methacrylic acid copolymer (for example, Eudragit FS30D); and enteric-soluble cellulosic polymer compounds such as cellulose acetate phthalate (for example, CAP, cellulose acetate phthalate), hypromellose phthalate (for example, HP-55), for example, hypromellose acetate succinate, polyvinyl acetate phthalate, and carboxymethylethyl cellulose.

Among these, methacrylic polymers are preferred, and specifically, for example, gastric-soluble methacrylic polymers such as a methyl methacrylate-butyl methacrylate-dimethylaminoethyl methacrylate copolymer (for example, aminoalkyl methacrylate copolymer E (Eudragit EPO or Eudragit E100)) and a methyl methacrylate-diethylaminoethyl methacrylate copolymer (Kollicoat Smartseal 30D); water-insoluble methacrylic polymers such as an ethyl acrylate-methyl methacrylate copolymer (for example, an ethyl acrylate-methyl methacrylate copolymer dispersion liquid (Eudragit NE30D)) and an ethyl acrylate-methyl methacrylate-ethyl methacrylate trimethylammonium chloride copolymer (for example, aminoalkyl methacrylate copolymer RS (Eudragit RS100, Eudragit RSPO, Eudragit RL, or Eudragit RLPO), and an aminoalkyl methacrylate copolymer RS aqueous dispersion liquid (Eudragit RS30D or Eudragit RL30D)); enteric-soluble methacrylic polymers such as a methacrylic acid-ethyl acrylate copolymer (for example, methacrylate copolymer LD (Eudragit L30D-55)), dry methacrylic acid copolymer LD (Eudragit L100-55)), a methacrylic acid-methyl methacrylate copolymer (for example, methacrylic acid copolymer L (Eudragit L100), methacrylic acid copolymer S (Eudragit S100)), and a methyl acrylate-methyl methacrylate-methacrylic acid copolymer (for example, Eudragit FS30D); and the like are preferred. In particular, a methyl methacrylate-diethylaminoethyl methacrylate copolymer, an ethyl acrylate-methyl methacrylate copolymer, and the like are preferred because the masking effect or the like is high and also the disintegration property is excellent.

The content of the polymer in the present invention varies depending on the kind of polymer, however, the polymer is generally contained in an amount of preferably 0.5 to 2000 parts by mass, more preferably 1 to 500 parts by mass, particularly preferably 5 to 200 parts by mass with respect to 100 parts by mass of the drug.

As the specific additive for coating the polymer-coated, granulated substance coated with a coating film of the above-mentioned polymer, one kind or a combination of two or more kinds selected from a metal stearate such as magnesium stearate, calcium stearate, aluminum stearate, or zinc stearate, stearic acid, a sucrose fatty acid ester, silicic acid, polyethylene glycol, talc, etc. can be exemplified. Among these, magnesium stearate, calcium stearate, stearic acid, a sucrose fatty acid ester, silicic acid, talc, etc. are preferred because the disintegration property improving effect is excellent, and magnesium stearate, calcium stearate, a sucrose fatty acid ester, and talc are more preferred.

In the present invention, besides the above-mentioned components, according to need, a component to be used in a general oral pharmaceutical preparation may be blended appropriately depending on the intended purpose.

Examples of the component to be used in a general oral pharmaceutical preparation include additives such as an excipient, a plasticizer, a binder, a disintegrating agent, a taste masking agent, a flavor, a fluidity improver, and a sweetener. These additives are described in PFSB/ELD Notification No. 1204-1 (Pharmaceutical Administration and Regulations), Japanese Pharmaceutical Excipients Directory 2007 (edited by Japan Pharmaceutical Excipients Council, Yakuji Nippo, Ltd.), and Eighth Edition Japan's Specifications and Standards for Food Additives (Japan Food Additives Association).

Examples of the excipient among the above-mentioned pharmaceutical additives include lactose, a starch, pregelatinized starch, crystalline cellulose, low-substituted hydroxypropyl cellulose, hydroxypropyl cellulose, refined white sugar, a sugar alcohol, light anhydrous silicic acid, calcium silicate, titanium oxide, and precipitated calcium carbonate. Among these excipients, one kind or two or more kinds can be used.

Examples of the plasticizer include triethyl citrate, glycerin, triacetin, propylene glycol, glyceryl monostearate, and a polyethylene glycol.

Examples of the binder include gelatin, gum Arabic powder, methyl cellulose, carboxymethyl cellulose, sodium carboxymethyl cellulose, hydroxypropyl cellulose, hypromellose, polyvinylpyrrolidone, polyvinyl alcohol, a polyvinyl alcohol-acrylic acid-methyl methacrylate copolymer, a polyvinyl alcohol-polyethylene glycol graft copolymer, pullulan, dextrin, carboxymethyl cellulose calcium, sodium carboxymethyl cellulose, shellac, a carboxyvinyl polymer, sodium carboxymethyl starch, carboxymethylethyl cellulose, and cellulose acetate phthalate. Among these binders, one kind or two or more kinds can be used.

Examples of the disintegrating agent include croscarmellose sodium, crospovidone, carmellose, carmellose calcium, carmellose sodium, sodium carboxymethyl starch, low-substituted hydroxypropyl cellulose, potato starch, cornstarch, and pregelatinized starch. Among these disintegrating agents, one kind or two or more kinds can be used.

Examples of the taste masking agent include malic acid, citric acid, and tartaric acid.

Examples of the flavor include an orange flavor, a spearmint flavor, a peppermint flavor, a yogurt flavor, and a lemon flavor.

Examples of the fluidity improver include hydrated silicon dioxide, light anhydrous silicic acid, talc, and magnesium aluminometasilicate.

Examples of the sweetener include aspartame, maltitol, saccharin, and a metal glycyrrhizinate.

In the production of the compression-molded preparation of the present invention, first, the above-mentioned drug and according to need, an additive such as an excipient are used and granulated according to a known granulation method such as a high-speed stirring granulation method or a fluidized bed granulation method, whereby a granulated substance containing the drug (hereinafter sometimes referred to as "drug-containing granulated substance") is prepared. For example, in the above-mentioned drug in the form of a powder, an excipient, a binder, or the like is mixed as needed, and water is added to the resulting mixed powder, followed by granulation by stirring granulation or the like, and further drying using a fluidized bed dryer or the like, whereby a drug-containing granulated substance is obtained.

Subsequently, the surface of this drug-containing granulated substance is coated with a coating film of the above-mentioned polymer, whereby a polymer-coated, granulated substance is obtained. The polymer coating film can be formed on the surface of the drug-containing granulated substance according to a conventional method, and for example, coating may be performed by spraying a coating liquid containing the above-mentioned polymer onto the drug-containing granulated substance in a fluidized bed.

As the coating liquid, for example, a solution or a dispersion liquid containing the above-mentioned polymer can be used, and as a solvent, water, a lower alcohol, or a mixed liquid of these, or the like is used. As the lower alcohol, a primary alcohol having 1 to 3 carbon atoms such as ethanol or isopropanol is exemplified. In the case where two or more kinds of polymers are used, these may be combined and dissolved or dispersed in a solvent, and the resulting material may be sprayed, or each of the respective polymers is separately dissolved or dispersed in a solvent, and the resulting materials may be sprayed a plurality of separate times . In the coating liquid, the above-mentioned additive may be blended as needed.

In the present invention, the coating with a polymer coating film means that the entire or a part of the surface of the drug-containing granulated substance is coated with a polymer coating film. From the viewpoint of expression of the function attributed to the polymer coating film such as a masking effect, coating efficiency with the specific additive, etc., it is preferred that the entire surface of the drug-containing granulated substance is coated.

The thus obtained polymer-coated, granulated substance may be further sized by sieving or the like. This polymer-coated, granulated substance has an average particle diameter of preferably about 20 to 1000 µm, more preferably from 50 to 700 µm. Incidentally, the average particle diameter of the polymer-coated, granulated substance is a value measured by mass and volume distributions.

Further, by coating the polymer-coated, granulated substance with the specific additive, the granules of the present invention can be obtained. In the coating with the specific additive, the polymer-coated, granulated substance and the specific additive in the form of particles may be mixed according to a conventional method, and for example, a common mixer such as a bohle container mixer, a V-type mixer, a ribbon blender, or a stirrer can be used. In the mixing, it is preferred that only the polymer-coated, granulated substance and the specific additive are mixed for obtaining a sufficient disintegration property improving effect. The coating amount of the specific additive is, for example, preferably from 0.01 to 5 parts by mass, more preferably from 0.15 to 0.25 parts by mass with respect to 100 parts by mass of the polymer-coated, granulated substance. In the present invention, the coating with the specific additive means that the specific additive in the form of particles is present in at least a part of the surface of the polymer coating film formed on the drug-containing granulated substance.

The granules of the present invention may be sized by sieving or the like as needed. The granules have an average particle diameter of preferably about 20 to 1000 µm, more preferably from 50 to 700 µm. Incidentally, the average particle diameter of the granules is a value measured by mass and volume distributions.

The compression-molded preparation of the present invention is produced by adding various additives to the granules as needed, followed by mixing and compression molding using a single punch tableting machine, a rotary tableting machine, or the like. As the additive, a fluidity improver can also be used. The content of the specific additive for coating the polymer-coated, granulated substance in the compression-molded preparation of the present invention is, for example, preferably from 0.01 to 5%, more preferably from 0.15 to 0.25%. Further, the pressure during the compression molding is, for example, preferably from 20 to 600 MPa, more preferably from 60 to 350 MPa, further more preferably from 170 to 300 MPa. When the pressure is within such a range, a preparation having a hardness free from practical problems and a favorable disintegration property is obtained. Further, the disintegration time is preferably within 1 minute, more preferably within 30 seconds. Incidentally, the disintegration time is a value measured according to the disintegration test method described in the Japanese Pharmacopoeia, Sixteenth Edition.

The thus obtained compression-molded preparation of the present invention uses a granulated substance coated with a coating film of a methacrylic polymer or the like, and therefore, has an excellent effect of masking the bitter taste or the like of a drug, etc., and also has a favorable disintegration property. In addition, even if a tableting pressure is increased, a variation in the tablet hardness or disintegration property is small, and a moderate harness and a favorable disintegration property are obtained within a wide tableting pressure range, and therefore, easy and stable production is possible under a condition that tableting trouble or the like hardly occurs. Further, the granule of the present invention exhibits an excellent disintegration property even if it is compression-molded, and therefore is suitable as a granule for use in a compression-molded preparation such as an orally disintegrating tablet, but is also utilized as a granule preparation, a capsule preparation obtained by filling a capsule with the granule, etc.

As particularly preferred embodiments of the compression-molded preparation of the present invention described above, the following preparation can be exemplified.

| | |
|---|---|
| Drug | 15 to 90 mass% |
| Methacrylic polymer | 1 to 50 mass% |
| Specific additive | 0.01 to 5 mass% |

### Examples

Next, the present invention will be more specifically described by showing Examples and Comparative Examples, however, the present invention is by no means limited thereto.

### Example 1

681 g of loxoprofen sodium hydrate (manufactured by Daiwa Pharmaceutical Co., Ltd.), 85 g of crystalline cellulose (manufactured by Asahi Kasei Chemicals Co., Ltd.), 200 g of low-substituted hydroxypropyl cellulose (manufactured by Shin-Etsu Chemical Co., Ltd.), 20 g of light anhydrous silicic acid (manufactured by Nippon Aerosil Co. Ltd.), and 14 g of polyvinyl alcohol (a partially saponified product) (manufactured by The Nippon Synthetic Chemical Industry Co., Ltd.) were mixed using a high-speed stirring-type mixing granulator. To this mixture, 250 g of purified water was added, followed by granulation. Subsequently, the granulated substance was dried using a fluidized bed dryer and sized using a sizing machine. 200 of this dried granulated substance was taken out, and in a fluidized bed, a coating liquid obtained by uniformly mixing 488 g of Kollicoat Smartseal 30D (solid content: 30%) (manufactured by BASF, Ltd.), 22 g of triethyl citrate (manufactured by Morimura Bros., Inc.), 7.3 g of light anhydrous silicic acid (manufactured by Nippon Aerosil Co. Ltd.), 4.4 g of sodium carboxymethyl cellulose (manufactured by DKS Co., Ltd.), and 595.3 g of purified water was sprayed onto the granulated substance until the increase in the mass of the granulated substance reached 30%, whereby a polymer-coated, granulated substance was obtained.

130 g of the polymer-coated, granulated substance and 0.65 g of magnesium stearate (manufactured by Taihei Chemical Industrial Co., Ltd.) were mixed for 10 minutes using a bohle container mixer, and thereafter, 26 g of cornstarch (manufactured by Nihon Cornstarch corporation), 81.9 g of crystalline cellulose (manufactured by Asahi Kasei Chemicals Co., Ltd.), 13 g of crospovidone (manufactured by BASF Japan Ltd.), 2.6 g of aspartame (manufactured by Ajinomoto Co., Inc.), 2.6 g of acesulfame potassium (manufactured by Kirin Kyowa Foods Company, Limited), 2.6 g of light anhydrous silicic acid (manufactured by Nippon Aerosil Co. Ltd.), and 0.65 g of magnesium stearate (manufactured by Taihei Chemical Industrial Co., Ltd.) were added thereto, followed by further mixing for 10 minutes. The resulting granules were tableted at a tableting pressure of 91, 123, 154, or 200 MPa using a rotary tableting machine so that the weight per tablet was 260 mg, whereby tablets each having a tablet diameter of 9 mm were obtained. With respect to each of the obtained tablets, the tablet hardness and the disintegration time were measured by the following methods.

### (Measurement Method for Tablet Hardness)

The tablet hardness in this description is a value measured using a load cell-type tablet hardness tester (product name: Portable Checker PC-30, manufactured by Okada Seiko Co., Ltd.).

### (Measurement Method for Disintegration Time)

The disintegration time in this description is a value measured according to the disintegration test method described in the Japanese Pharmacopoeia, Sixteenth Edition. As the test solution, purified water specified in the Japanese Pharmacopoeia was used, and the disintegration time was measured without using a disk.

### Example 2

130 g of the polymer-coated, granulated substance obtained in Example 1 and 0.325 g of magnesium stearate (manufactured by Taihei Chemical Industrial Co., Ltd.) were mixed for 10 minutes using a bohle container mixer, and thereafter, 26 g of cornstarch (manufactured by Nihon Cornstarch corporation), 81.9 g of crystalline cellulose (manufactured by Asahi Kasei Chemicals Co., Ltd.), 13 g of crospovidone (manufactured by BASF Japan Ltd.), 2.6 g of aspartame (manufactured by Ajinomoto Co., Inc.), 2.6 g of acesulfame potassium (manufactured by Kirin Kyowa Foods Company, Limited), 2.6 g of light anhydrous silicic acid (manufactured by Nippon Aerosil Co. Ltd.), and 0.975 g of magnesium stearate (manufactured by Taihei Chemical Industrial Co., Ltd.) were added thereto, followed by further mixing for 10 minutes. The resulting granules were tableted at a tableting pressure of 91, 123, 154, or 200 MPa using a rotary tableting machine so that the weight per tablet was 260 mg, whereby tablets each having a tablet diameter of 9 mm were obtained. With respect to each of the obtained tablets, the tablet hardness and the disintegration time were measured in the same manner as in Example 1.

### Example 3

130 g of the polymer-coated, granulated substance obtained in Example 1 and 0.13 g of magnesium stearate (manufactured by Taihei Chemical Industrial Co., Ltd.) were mixed for 10 minutes using a bohle container mixer, and thereafter, 26 g of cornstarch (manufactured by Nihon Cornstarch corporation), 81.9 g of crystalline cellulose (manufactured by Asahi Kasei Chemicals Co., Ltd.), 13 g of crospovidone (manufactured by BASF Japan Ltd.), 2.6 g of aspartame (manufactured by Ajinomoto Co., Inc.), 2.6 g of acesulfame potassium (manufactured by Kirin Kyowa Foods Company, Limited), 2.6 g of light anhydrous silicic acid (manufactured by Nippon Aerosil Co. Ltd.), and 1.17 g of magnesium stearate (manufactured by Taihei Chemical Industrial Co., Ltd.) were added thereto, followed by further mixing for 10 minutes. The resulting granules were tableted at a tableting pressure of 91, 123, 154, or 200 MPa using a rotary tableting machine so that the weight per tablet was 260 mg, whereby tablets each having a tablet diameter of 9 mm were obtained. With respect to each of the obtained tablets, the tablet hardness and the disintegration time were measured in the same manner as in Example 1.

### Comparative Example 1

To 130 g of the polymer-coated, granulated substance obtained in Example 1, 26 g of cornstarch (manufactured by Nihon Cornstarch corporation), 81.9 g of crystalline cellulose (manufactured by Asahi Kasei Chemicals Co., Ltd.), 13 g of crospovidone (manufactured by BASF Japan Ltd.), 2.6 g of aspartame (manufactured by Ajinomoto Co., Inc.), 2.6 g of acesulfame potassium (manufactured by Kirin Kyowa Foods Company, Limited), 2.6 g of light anhydrous silicic acid (manufactured by Nippon Aerosil Co. Ltd.), and 1.3 g of magnesium stearate (manufactured by Taihei Chemical Industrial Co., Ltd.) were added, followed by further mixing for 10 minutes. The resulting granules were tableted at a tableting pressure of 91, 123, 154, or 200 MPa using a rotary tableting machine so that the weight per tablet was 260 mg, whereby tablets each having a tablet diameter of 9 mm were obtained. With respect to each of the obtained tablets, the tablet hardness and the disintegration time were measured in the same manner as in Example 1.

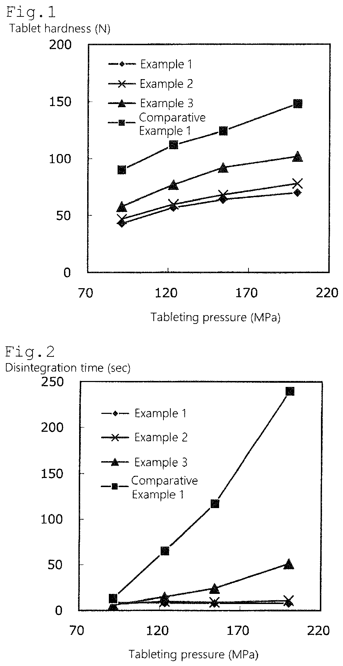

With respect to the tablets of Examples 1 to 3 and Comparative Example 1, the tablet hardness are shown in Table 1 and FIG. 1, and the disintegration time are shown in Table 2 and FIG. 2.

**[Table 1]**

| (N) | | | | |
|---|---|---|---|---|
| Tableting pressure (MPa) | Example 1 | Example 2 | Example 3 | Comparative Example 1 |
| 91 | 43 | 47 | 58 | 90 |
| 123 | 57 | 60 | 77 | 112 |
| 154 | 64 | 68 | 92 | 124 |
| 200 | 70 | 78 | 102 | 148 |

**[Table 2]**

| (sec) | | | | |
|---|---|---|---|---|
| Tableting pressure (MPa) | Example 1 | Example 2 | Example 3 | Comparative Example 1 |
| 91 | 9 | 7 | 6 | 13 |
| 123 | 8 | 10 | 15 | 65 |
| 154 | 8 | 9 | 24 | 117 |
| 200 | 8 | 11 | 51 | 240 |

### Example 4

320 g of purified water was added to 600 g of ibuprofen sodium (manufactured by BASF, Ltd.), 330 g of crystalline cellulose (manufactured by Asahi Kasei Chemicals Co., Ltd.) , 24 g of polyvinyl acetal diethylamino acetate (manufactured by Mitsubishi-Chemical Foods Corporation), 39.9 g of Eudragit L30D-55 (solid content: 30%) (manufactured by Evonik Industries), 12 g of DL-malic acid (manufactured by Fuso Chemical Co., Ltd.), and 12 g of polyvinyl alcohol (a partially saponified product) (manufactured by The Nippon Synthetic Chemical Industry Co., Ltd.), and the resulting mixture was granulated using a high-speed stirring-type mixing granulator. Subsequently, the granulated substance was dried using a fluidized bed dryer and sized using a sizing machine. 330 g of this dried granulated substance was taken out, and in a fluidized bed, a coating liquid obtained by uniformly mixing 100 g of methacrylic acid copolymer LD (solid content: 30%) (manufactured by Evonik Industries), 3 g of triethyl citrate (manufactured by Morimura Bros., Inc.), 3 g of light anhydrous silicic acid (manufactured by Nippon Aerosil Co. Ltd.), and 197 g of purified water was sprayed onto the granulated substance until the increase in the mass of the granulated substance reached 10%, whereby a polymer-coated, granulated substance was obtained. 366 g of the polymer-coated, granulated substance and 1.83 g of magnesium stearate (manufactured by Taihei Chemical Industrial Co., Ltd.) were mixed for 10 minutes using a bohle container mixer, and thereafter, 120 g of crystalline cellulose (manufactured by Asahi Kasei Chemicals Co., Ltd.), 76 g of cornstarch (manufactured by Nihon Cornstarch corporation), 6 g of acesulfame potassium (manufactured by Kirin Kyowa Foods Company, Limited), 6 g of aspartame (manufactured by Ajinomoto Co., Inc.), and 6 g of light anhydrous silicic acid (manufactured by Nippon Aerosil Co. Ltd.) were added thereto, followed by further mixing for 10 minutes. The resulting granules were tableted at a tableting pressure of 87, 104, 121, or 157 MPa using a rotary tableting machine so that the weight per tablet was 581.8 mg, whereby tablets each having a tablet diameter of 12 mm were obtained. With respect to each of the tablets, the tablet hardness and the disintegration time were measured in the same manner as in Example 1.

### Comparative Example 2

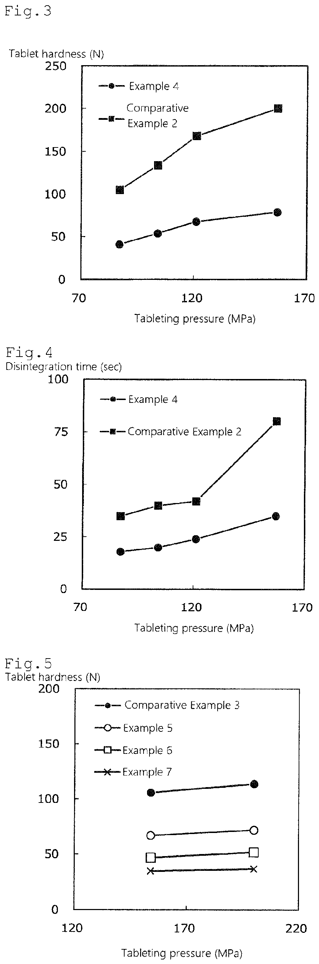

To 130 g of the polymer-coated, granulated substance obtained in the above Example 4, 120 g of crystalline cellulose (manufactured by Asahi Kasei Chemicals Co., Ltd.), 76 g of cornstarch (manufactured by Nihon Cornstarch corporation), 6 g of acesulfame potassium (manufactured by Kirin Kyowa Foods Company, Limited), 6 g of aspartame (manufactured by Ajinomoto Co., Inc.), and 6 g of light anhydrous silicic acid (manufactured by Nippon Aerosil Co. Ltd.) were added thereto, followed by further mixing for 10 minutes. The resulting granules were tableted at a tableting pressure of 87, 104, 121, or 157 MPa using a rotary tableting machine so that the weight per tablet was 581.8 mg, whereby tablets each having a tablet diameter of 12 mm were obtained. With respect to each of the tablets, the tablet hardness and the disintegration time were measured in the same manner as in Example 1. With respect to Example 4 and Comparative Example 2, the tablet hardness is shown in Table 3 and FIG. 3, and the disintegration time is shown in Table 4 and FIG. 4.

**[Table 3]**

| (N) | | |
|---|---|---|
| Tableting pressure (MPa) | Example 4 | Comparative Example 2 |
| 87 | 41 | 105 |
| 104 | 54 | 134 |
| 121 | 68 | 168 |
| 157 | 79 | 200 |

**[Table 4]**

| (sec) | | |
|---|---|---|
| Tableting pressure (MPa) | Example 4 | Comparative Example 2 |
| 87 | 18 | 35 |
| 104 | 20 | 40 |
| 121 | 24 | 42 |
| 157 | 35 | 80 |

### Example 5

681 g of loxoprofen sodium hydrate (manufactured by Daiwa Pharmaceutical Co., Ltd.), 85 g of crystalline cellulose (manufactured by Asahi Kasei Chemicals Co., Ltd.), 200 g of low-substituted hydroxypropyl cellulose (manufactured by Shin-Etsu Chemical Co., Ltd.), 20 g of light anhydrous silicic acid (manufactured by Nippon Aerosil Co. Ltd.), and 14 g of polyvinyl alcohol (a partially saponified product) (manufactured by The Nippon Synthetic Chemical Industry Co., Ltd.) were mixed using a high-speed stirring-type mixing granulator. To this mixture, 250 g of purified water was added, followed by granulation. Subsequently, the granulated substance was dried using a fluidized bed dryer and sized using a sizing machine. 200 g of this dried granulated substance was taken out, and in a fluidized bed, a coating liquid obtained by uniformly mixing 488 g of Kollicoat Smartseal 30D (solid content: 30%) (manufactured by BASF, Ltd.), 22 g of triethyl citrate (manufactured by Morimura Bros., Inc.), 7.3 g of light anhydrous silicic acid (manufactured by Nippon Aerosil Co. Ltd.), 4.4 g of sodium carboxymethyl cellulose (manufactured by DKS Co., Ltd.), and 595.3 g of purified water was sprayed onto the granulated substance until the increase in the mass of the granulated substance reached 30%, whereby a polymer-coated, granulated substance was obtained.

130 g of the polymer-coated, granulated substance and 1.30 g of magnesium stearate (manufactured by Taihei Chemical Industrial Co., Ltd.) were mixed for 2 minutes in a polyethylene bag, and thereafter, 26 g of cornstarch (manufactured by Nihon Cornstarch corporation), 87.1 g of crystalline cellulose (manufactured by Asahi Kasei Chemicals Co., Ltd.), 13 g of crospovidone (manufactured by BASF Ltd.), and 2.6 g of light anhydrous silicic acid (manufactured by Nippon Aerosil Co. Ltd.) were added thereto, followed by further mixing for 2 minutes. The resulting granules were tableted at a tableting pressure of 154 or 200 MPa using a rotary tableting machine so that the weight per tablet was 260 mg, whereby tablets each having a tablet diameter of 9 mm were obtained. With respect to each of the obtained tablets, the tablet hardness and the disintegration time were measured in the same manner as in Example 1.

### Example 6

130 g of the polymer-coated, granulated substance obtained in Example 5 and 3.90 g of magnesium stearate (manufactured by Taihei Chemical Industrial Co., Ltd.) were mixed for 2 minutes in a polyethylene bag, and thereafter, 26 g of cornstarch (manufactured by Nihon Cornstarch corporation), 87.1 g of crystalline cellulose (manufactured by Asahi Kasei Chemicals Co., Ltd.), 13 g of crospovidone (manufactured by BASF Ltd.), and 2.6 g of light anhydrous silicic acid (manufactured by Nippon Aerosil Co. Ltd.) were added thereto, followed by further mixing for 2 minutes. The resulting granules were tableted at a tableting pressure of 154 or 200 MPa using a rotary tableting machine so that the weight per tablet was 262.6 mg, whereby tablets each having a tablet diameter of 9 mm were obtained. With respect to each of the obtained tablets, the tablet hardness and the disintegration time were measured in the same manner as in Example 1.

### Example 7

130 g of the polymer-coated, granulated substance obtained in Example 5 and 6.5 g of magnesium stearate (manufactured by Taihei Chemical Industrial Co., Ltd.) were mixed for 2 minutes in a polyethylene bag, and thereafter, 26 g of cornstarch (manufactured by Nihon Cornstarch corporation), 87.1 g of crystalline cellulose (manufactured by Asahi Kasei Chemicals Co., Ltd.), 13 g of crospovidone (manufactured by BASF Ltd.), and 2.6 g of light anhydrous silicic acid (manufactured by Nippon Aerosil Co. Ltd.) were added thereto, followed by further mixing for 2 minutes. The resulting granules were tableted at a tableting pressure of 154 or 200 MPa using a rotary tableting machine so that the weight per tablet was 265.2 mg, whereby tablets each having a tablet diameter of 9 mm were obtained. With respect to each of the obtained tablets, the tablet hardness and the disintegration time were measured in the same manner as in Example 1.

### Comparative Example 3

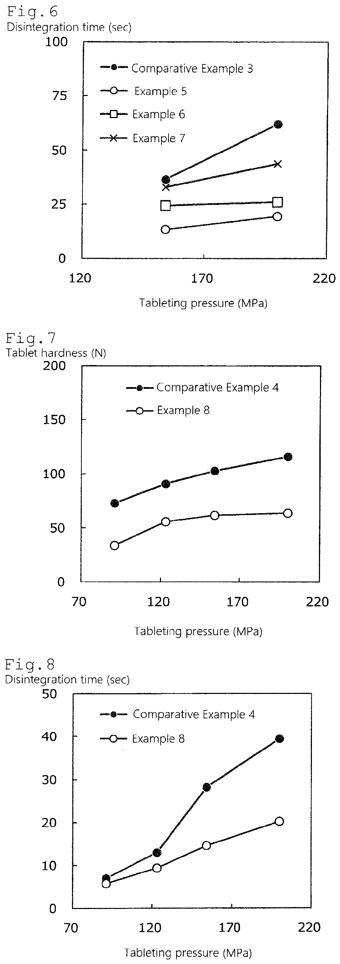

130 g of the polymer-coated, granulated substance obtained in Example 5 was mixed with 26 g of cornstarch (manufactured by Nihon Cornstarch corporation), 87.1 g of crystalline cellulose (manufactured by Asahi Kasei Chemicals Co., Ltd.), 13 g of crospovidone (manufactured by BASF Ltd.), and 2.6 g of light anhydrous silicic acid (manufactured by Nippon Aerosil Co. Ltd.) for 2 minutes in a polyethylene bag. The resulting granules were tableted at a tableting pressure of 154 or 200 MPa using a rotary tableting machine so that the weight per tablet was 260 mg, whereby tablets each having a tablet diameter of 9 mm were obtained. With respect to each of the obtained tablets, the tablet hardness and the disintegration time were measured in the same manner as in Example 1. With respect to Examples 5 to 7 and Comparative Example 3, the tablet hardness with respect to the addition amount of magnesium stearate at each tableting pressure is shown in Table 5 and FIG. 5, and the disintegration time is shown in Table 6 and FIG. 6.

With respect to the tablets of Examples 5 to 7 and Comparative Example 3, the tablet hardness are shown in Table 5 and FIG. 5, and the disintegration time are shown in Table 6 and FIG. 6.

**[Table 5]**

| (N) | | | | |
|---|---|---|---|---|
| Tableting pressure (MPa) | Comparative Example 3 | Example 5 | Example 6 | Example 7 |
| 154 | 106 | 67 | 47 | 35 |
| 200 | 114 | 72 | 52 | 37 |

**[Table 6]**

| (sec) | | | | |
|---|---|---|---|---|
| Tableting pressure (MPa) | Comparative Example 3 | Example 5 | Example 6 | Example 7 |
| 154 | 37 | 13 | 24 | 33 |
| 200 | 62 | 19 | 26 | 44 |

### Example 8

681 g of loxoprofen sodium hydrate (manufactured by Daiwa Pharmaceutical Co., Ltd.), 85 g of crystalline cellulose (manufactured by Asahi Kasei Chemicals Co., Ltd.), 200 g of low-substituted hydroxypropyl cellulose (manufactured by Shin-Etsu Chemical Co., Ltd.), 20 g of light anhydrous silicic acid (manufactured by Nippon Aerosil Co. Ltd.), and 14 g of polyvinyl alcohol (a partially saponified product) (manufactured by The Nippon Synthetic Chemical Industry Co., Ltd.) were mixed using a high-speed stirring-type mixing granulator. To this mixture, 250 g of purified water was added, followed by granulation. Subsequently, the granulated substance was dried using a fluidized bed dryer and sized using a sizing machine. 200 g of this dried granulated substance was taken out, and in a fluidized bed, a coating liquid obtained by uniformly mixing 488 g of Kollicoat Smartseal 30D (solid content: 30%) (manufactured by BASF, Ltd.), 22 g of triethyl citrate (manufactured by Morimura Bros., Inc.), 7.3 g of light anhydrous silicic acid (manufactured by Nippon Aerosil Co. Ltd.), 4.4 g of sodium carboxymethyl cellulose (manufactured by DKS Co., Ltd.), and 595.3 g of purified water was sprayed onto the granulated substance until the increase in the mass of the granulated substance reached 30%, whereby a polymer-coated, granulated substance was obtained.

130 g of the polymer-coated, granulated substance and 1.30 g of magnesium stearate (manufactured by Taihei Chemical Industrial Co., Ltd.) were mixed for 2 minutes in a polyethylene bag, and thereafter, 26 g of cornstarch (manufactured by Nihon Cornstarch corporation), 87.1 g of crystalline cellulose (manufactured by Asahi Kasei Chemicals Co., Ltd.), 13 g of crospovidone (manufactured by BASF Japan Ltd.), and 2.6 g of light anhydrous silicic acid (manufactured by Nippon Aerosil Co. Ltd.) were added thereto, followed by further mixing for 2 minutes. The resulting granules were tableted at a tableting pressure of 91, 123, 154, or 200 MPa using a rotary tableting machine so that the weight per tablet was 260 mg, whereby tablets each having a tablet diameter of 9 mm were obtained. With respect to each of the tablets, the tablet hardness and the disintegration time were measured in the same manner as in Example 1.

### Comparative Example 4

130 g of the polymer-coated, granulated substance obtained in Example 8 was mixed with 26 g of cornstarch (manufactured by Nihon Cornstarch corporation), 87.1 g of crystalline cellulose (manufactured by Asahi Kasei Chemicals Co., Ltd.), 13 g of crospovidone (manufactured by BASF Ltd.), 1.30 g of magnesium stearate (manufactured by Taihei Chemical Industrial Co., Ltd.), and 2.6 g of light anhydrous silicic acid (manufactured by Nippon Aerosil Co. Ltd.) for 2 minutes in a polyethylene bag. The resulting granules were tableted at a tableting pressure of 91, 123, 154, or 200 MPa using a rotary tableting machine so that the weight per tablet was 260 mg, whereby tablets each having a tablet diameter of 9 mm were obtained. With respect to each of the obtained tablets, the tablet hardness and the disintegration time were measured in the same manner as in Example 1. With respect to Example 8 and Comparative Example 4, the tablet hardness is shown in Table 7 and FIG. 7, and the disintegration time is shown in Table 8 and FIG. 8.

**[Table 7]**

| (N) | | |
|---|---|---|
| Tableting pressure (MPa) | Example 8 | Comparative Example 4 |
| 91 | 34 | 73 |
| 123 | 56 | 91 |
| 154 | 62 | 103 |
| 200 | 64 | 116 |

**[Table 8]**

| (sec) | | |
|---|---|---|
| Tableting pressure (MPa) | Example 8 | Comparative Example 4 |
| 91 | 6 | 7 |
| 123 | 9 | 13 |
| 154 | 15 | 28 |
| 200 | 20 | 39 |

### Example 9

130 g of the polymer-coated, granulated substance obtained in Example 8 and 3.9 g of magnesium stearate (manufactured by Taihei Chemical Industrial Co., Ltd.) were mixed for 2 minutes in a polyethylene bag, and thereafter, 26 g of cornstarch (manufactured by Nihon Cornstarch corporation), 87.1 g of crystalline cellulose (manufactured by Asahi Kasei Chemicals Co., Ltd.), 13 g of crospovidone (manufactured by BASF Ltd.), and 2.6 g of light anhydrous silicic acid (manufactured by Nippon Aerosil Co. Ltd.) were added thereto, followed by further mixing for 2 minutes. The resulting granules were tableted at a tableting pressure of 91, 123, 154, or 200 MPa using a rotary tableting machine so that the weight per tablet was 262.6 mg, whereby tablets each having a tablet diameter of 9 mm were obtained. With respect to each of the obtained tablets, the tablet hardness and the disintegration time were measured in the same manner as in Example 1.

### Comparative Example 5

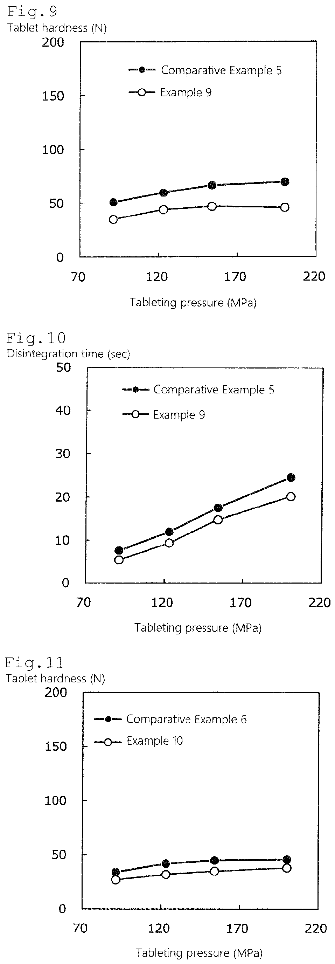

130 g of the polymer-coated, granulated substance obtained in Example 8 was mixed with 26 g of cornstarch (manufactured by Nihon Cornstarch corporation), 87.1 g of crystalline cellulose (manufactured by Asahi Kasei Chemicals Co., Ltd.), 13 g of crospovidone (manufactured by BASF Ltd.), 3.9 g of magnesium stearate (manufactured by Taihei Chemical Industrial Co., Ltd.), and 2.6 g of light anhydrous silicic acid (manufactured by Nippon Aerosil Co. Ltd.) for 2 minutes in a polyethylene bag. The resulting granules were tableted at a tableting pressure of 91, 123, 154, or 200 MPa using a rotary tableting machine so that the weight per tablet was 260 mg, whereby tablets each having a tablet diameter of 9 mm were obtained. With respect to each of the obtained tablets, the tablet hardness and the disintegration time were measured in the same manner as in Example 1. With respect to Example 9 and Comparative Example 5, the tablet hardness is shown in Table 9 and FIG. 9, and the disintegration time is shown in Table 10 and FIG. 10.

**[Table 9]**

| (N) | | |
|---|---|---|
| Tableting pressure (MPa) | Example 9 | Comparative Example 5 |
| 91 | 35 | 51 |
| 123 | 44 | 60 |
| 154 | 47 | 67 |
| 200 | 46 | 70 |

**[Table 10]**

| (sec) | | |
|---|---|---|
| Tableting pressure (MPa) | Example 9 | Comparative Example 5 |
| 91 | 5 | 8 |
| 123 | 9 | 12 |
| 154 | 15 | 18 |
| 200 | 20 | 25 |

### Example 10

130 g of the polymer-coated, granulated substance obtained in Example 8 and 6.5 g of magnesium stearate (manufactured by Taihei Chemical Industrial Co., Ltd.) were mixed for 2 minutes in a polyethylene bag, and thereafter, 26 g of cornstarch (manufactured by Nihon Cornstarch corporation), 87.1 g of crystalline cellulose (manufactured by Asahi Kasei Chemicals Co., Ltd.), 13 g of crospovidone (manufactured by BASF Ltd.), and 2.6 g of light anhydrous silicic acid (manufactured by Nippon Aerosil Co. Ltd.) were added thereto, followed by further mixing for 2 minutes. The resulting granules were tableted at a tableting pressure of 91, 123, 154, or 200 MPa using a rotary tableting machine so that the weight per tablet was 265.2 mg, whereby tablets each having a tablet diameter of 9 mm were obtained. With respect to each of the obtained tablets, the tablet hardness and the disintegration time were measured in the same manner as in Example 1.

### Comparative Example 6

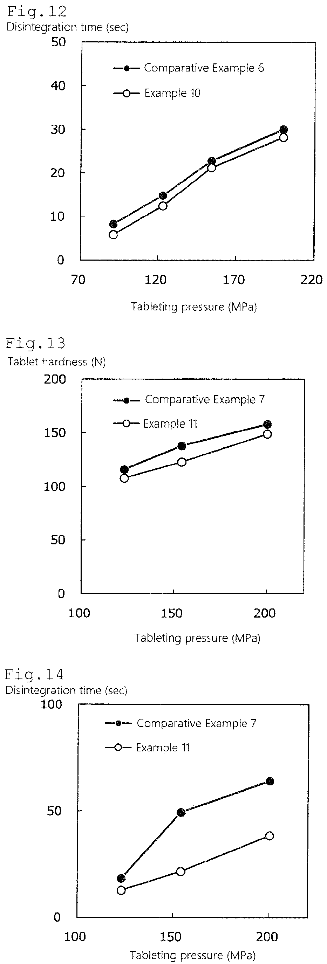

130 g of the polymer-coated, granulated substance obtained in Example 8 was mixed with 26 g of cornstarch (manufactured by Nihon Cornstarch corporation), 87.1 g of crystalline cellulose (manufactured by Asahi Kasei Chemicals Co., Ltd.), 13 g of crospovidone (manufactured by BASF Ltd.), 6.5 g of magnesium stearate (manufactured by Taihei Chemical Industrial Co., Ltd.), and 2.6 g of light anhydrous silicic acid (manufactured by Nippon Aerosil Co. Ltd.) for 2 minutes in a polyethylene bag. The resulting granules were tableted at a tableting pressure of 91, 123, 154, or 200 MPa using a rotary tableting machine so that the weight per tablet was 260 mg, whereby tablets each having a tablet diameter of 9 mm were obtained. With respect to each of the obtained tablets, the tablet hardness and the disintegration time were measured in the same manner as in Example 1. With respect to Example 10 and Comparative Example 6, the tablet hardness is shown in Table 11 and FIG. 11, and the disintegration time is shown in Table 12 and FIG. 12.

**[Table 11]**

| (N) | | |
|---|---|---|
| Tableting pressure (MPa) | Example 10 | Comparative Example 6 |
| 91 | 27 | 34 |
| 123 | 32 | 42 |
| 154 | 35 | 45 |
| 200 | 38 | 46 |

**[Table 12]**

| (sec) | | |
|---|---|---|
| Tableting pressure (MPa) | Example 10 | Comparative Example 6 |
| 91 | 6 | 8 |
| 123 | 12 | 15 |
| 154 | 21 | 23 |
| 200 | 28 | 30 |

### Example 11

681 g of loxoprofen sodium hydrate (manufactured by Daiwa Pharmaceutical Co., Ltd.), 85 g of crystalline cellulose (manufactured by Asahi Kasei Chemicals Co., Ltd.), 200 g of low-substituted hydroxypropyl cellulose (manufactured by Shin-Etsu Chemical Co., Ltd.), 20 g of light anhydrous silicic acid (manufactured by Nippon Aerosil Co. Ltd.), and 14 g of polyvinyl alcohol (a partially saponified product) (manufactured by The Nippon Synthetic Chemical Industry Co., Ltd.) were mixed using a high-speed stirring-type mixing granulator. To this mixture, 250 g of purified water was added, followed by granulation. Subsequently, the granulated substance was dried using a fluidized bed dryer and sized using a sizing machine. 200 g of this dried granulated substance was taken out, and in a fluidized bed, a coating liquid obtained by uniformly mixing 488 g of Kollicoat Smartseal 30D (solid content: 30%) (manufactured by BASF, Ltd.), 22 g of triethyl citrate (manufactured by Morimura Bros., Inc.), 7.3 g of light anhydrous silicic acid (manufactured by Nippon Aerosil Co. Ltd.), 4.4 g of sodium carboxymethyl cellulose (manufactured by DKS Co., Ltd.), and 595.3 g of purified water was sprayed onto the granulated substance until the increase in the mass of the granulated substance reached 30%, whereby a polymer-coated, granulated substance was obtained.

130 g of the polymer-coated, granulated substance and 1.30 g of talc (manufactured by Fuji Talc Industrial Co., Ltd.) were mixed for 2 minutes in a polyethylene bag, and thereafter, 26 g of cornstarch (manufactured by Nihon Cornstarch corporation), 87.1 g of crystalline cellulose (manufactured by Asahi Kasei Chemicals Co., Ltd.), 13 g of crospovidone (manufactured by BASF Japan Ltd.), and 2.6 g of light anhydrous silicic acid (manufactured by Nippon Aerosil Co. Ltd.) were added thereto, followed by further mixing for 2 minutes. The resulting granules were tableted at a tableting pressure of 123, 154, or 200 MPa using a rotary tableting machine so that the weight per tablet was 260 mg, whereby tablets each having a tablet diameter of 9 mm were obtained. With respect to each of the obtained tablets, the tablet hardness and the disintegration time were measured in the same manner as in Example 1.

### Comparative Example 7

130 g of the polymer-coated, granulated substance obtained in Example 11 was mixed with 26 g of cornstarch (manufactured by Nihon Cornstarch corporation), 87.1 g of crystalline cellulose (manufactured by Asahi Kasei Chemicals Co., Ltd.), 13 g of crospovidone (manufactured by BASF Ltd.), 1.3 g of talc (manufactured by Fuji Talc Industrial Co., Ltd.), and 2.6 g of light anhydrous silicic acid (manufactured by Nippon Aerosil Co. Ltd.) for 2 minutes in a polyethylene bag. The resulting granules were tableted at a tableting pressure of 123, 154, or 200 MPa using a rotary tableting machine so that the weight per tablet was 260 mg, whereby tablets each having a tablet diameter of 9 mm were obtained. With respect to each of the obtained tablets, the tablet hardness and the disintegration time were measured in the same manner as in Example 1. With respect to Example 11 and Comparative Example 7, the tablet hardness is shown in Table 13 and FIG. 13, and the disintegration time is shown in Table 14 and FIG. 14.

**[Table 13]**

| (N) | | |
|---|---|---|
| Tableting pressure (MPa) | Example 11 | Comparative Example 7 |
| 123 | 108 | 116 |
| 154 | 123 | 138 |
| 200 | 149 | 158 |

**[Table 14]**

| (sec) | | |
|---|---|---|
| Tableting pressure (MPa) | Example 11 | Comparative Example 7 |
| 123 | 13 | 18 |
| 154 | 22 | 49 |
| 200 | 38 | 64 |

### Example 12

Tablets were prepared in the same manner as in Example 11 except that 1.3 g of talc (manufactured by Fuji Talc Industrial Co., Ltd.) was replaced with 1.3 g of calcium stearate (manufactured by Taihei Chemical Industrial Co., Ltd.). With respect to each of the obtained tablets, the tablet hardness and the disintegration time were measured in the same manner as in Example 1.

### Comparative Example 8

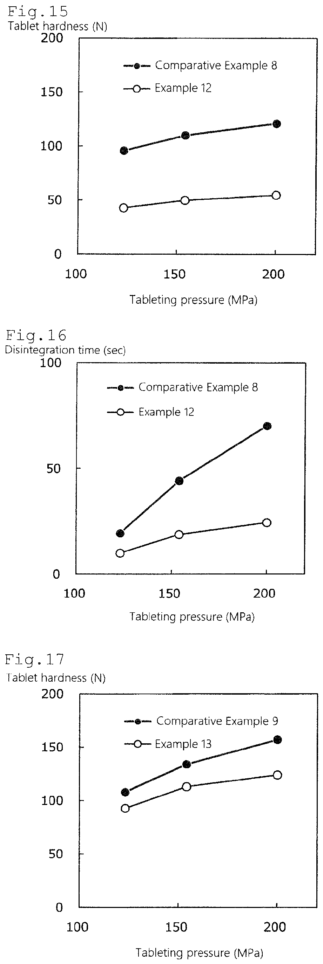

130 g of the polymer-coated, granulated substance obtained in Example 11 was mixed with 26 g of cornstarch (manufactured by Nihon Cornstarch corporation), 87.1 g of crystalline cellulose (manufactured by Asahi Kasei Chemicals Co., Ltd.), 13 g of crospovidone (manufactured by BASF Ltd.), 1.30 g of calcium stearate (manufactured by Taihei Chemical Industrial Co., Ltd.), and 2.6 g of light anhydrous silicic acid (manufactured by Nippon Aerosil Co. Ltd.) for 2 minutes in a polyethylene bag. The resulting granules were tableted at a tableting pressure of 123, 154, or 200 MPa using a rotary tableting machine so that the weight per tablet was 260 mg, whereby tablets each having a tablet diameter of 9 mm were obtained. With respect to each of the obtained tablets, the tablet hardness and the disintegration time were measured in the same manner as in Example 1. With respect to Example 12 and Comparative Example 8, the tablet hardness is shown in Table 15 and FIG. 15, and the disintegration time is shown in Table 16 and FIG. 16.

**[Table 15]**

| (N) | | |
|---|---|---|
| Tableting pressure (MPa) | Example 12 | Comparative Example 8 |
| 123 | 43 | 96 |
| 154 | 50 | 110 |
| 200 | 55 | 121 |

**[Table 16]**

| (sec) | | |
|---|---|---|
| Tableting pressure (MPa) | Example 12 | Comparative Example 8 |
| 123 | 10 | 19 |
| 154 | 19 | 44 |
| 200 | 24 | 70 |

### Example 13

Tablets were prepared in the same manner as in Example 11 except that talc (manufactured by Fuji Talc Industrial Co., Ltd.) was replaced with a sucrose fatty acid ester (manufactured by Mitsubishi-Chemical Foods Corporation). With respect to each of the obtained tablets, the tablet hardness and the disintegration time were measured in the same manner as in Example 1.

### Comparative Example 9

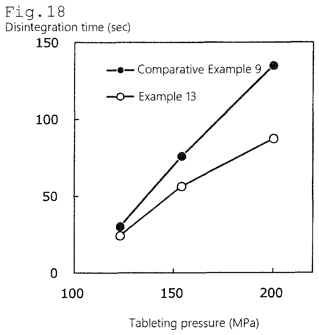

130 g of the polymer-coated, granulated substance obtained in Example 11 was mixed with 26 g of cornstarch (manufactured by Nihon Cornstarch corporation), 87.1 g of crystalline cellulose (manufactured by Asahi Kasei Chemicals Co., Ltd.), 13 g of crospovidone (manufactured by BASF Ltd.), 1.3 g of a sucrose fatty acid ester (manufactured by Mitsubishi-Chemical Foods Corporation), and 2.6 g of light anhydrous silicic acid (manufactured by Nippon Aerosil Co. Ltd.) for 2 minutes in a polyethylene bag. The resulting granules were tableted at a tableting pressure of 123, 154, or 200 MPa using a rotary tableting machine so that the weight per tablet was 260 mg, whereby tablets each having a tablet diameter of 9 mm were obtained. With respect to each of the obtained tablets, the tablet hardness and the disintegration time were measured in the same manner as in Example 1. With respect to Example 10 and Comparative Example 9, the tablet hardness is shown in Table 17 and FIG. 17, and the disintegration time is shown in Table 18 and FIG. 18.

**[Table 17]**

| (N) | | |
|---|---|---|
| Tableting pressure (MPa) | Example 13 | Comparative Example 9 |
| 123 | 93 | 108 |
| 154 | 113 | 134 |
| 200 | 124 | 157 |

**[Table 18]**

| (sec) | | |
|---|---|---|
| Tableting pressure (MPa) | Example 13 | Comparative Example 9 |
| 123 | 24 | 30 |
| 154 | 56 | 76 |
| 200 | 87 | 135 |

As shown in the above Tables 1 to 18 and FIGS. 1 to 18, it is clear that the disintegration time of the compression-molded preparations using the granules coated with the specific additive is shortened as compared with the compression-molded preparations including uncoated granules.

### Industrial Applicability

The compression-molded preparation using the granules of the present invention has an excellent effect of masking the bitter taste or the like of a drug having an unpleasant taste, etc., and also has a favorable disintegration property, and therefore, can be favorably utilized as an orally disintegrating tablet, etc.

## Claims

1. A compression-molded preparation, comprising granules obtained by coating a polymer-coated, granulated substance, in which a granulated substance containing a drug is coated with a polymer coating film, with one kind or two or more kinds of additives selected from the group consisting of a metal stearate, stearic acid, a sucrose fatty acid ester, talc, and silicic acid.

2. The compression-molded preparation according to claim 1, wherein the additive is magnesium stearate, calcium stearate, a sucrose fatty acid ester, or talc.

3. The compression-molded preparation according to claim 1 or 2, wherein the drug is a drug having an unpleasant taste.

4. The compression-molded preparation according to claim 3, wherein the drug having an unpleasant taste is one kind or two or more kinds selected from the group consisting of loxoprofen sodium, ibuprofen sodium, diclofenac potassium, diclofenac sodium, and naproxen sodium, or hydrates thereof.

5. The compression-molded preparation according to any one of claims 1 to 4, wherein the polymer is a methacrylic polymer.

6. The compression-molded preparation according to claim 5, wherein the methacrylic polymer is one kind or two or more kinds selected from the group consisting of a methyl methacrylate-diethylaminoethyl methacrylate copolymer, an ethyl acrylate-methyl methacrylate copolymer, a methacrylic acid-ethyl acrylate copolymer, and a methyl acrylate-methyl methacrylate-methacrylic acid copolymer.

7. The compression-molded preparation according to any one of claims 1 to 6, wherein the content of the polymer is from 0.5 to 2000 parts by mass with respect to 100 parts by mass of the drug.

8. The compression-molded preparation according to any one of claims 1 to 7, wherein the content of the additive for coating the polymer-coated, granulated substance is from 0.01 to 5 parts by mass with respect to 100 parts by mass of the polymer-coated, granulated substance.

9. The compression-molded preparation according to any one of claims 1 to 8, wherein the compression-molded preparation is an orally disintegrating tablet.

10. A method for producing a compression-molded preparation, **characterized in that** a polymer-coated, granulated substance is obtained by coating a granulated substance containing a drug with a polymer coating film, and then, granules obtained by coating the polymer-coated, granulated substance with one kind or two or more kinds of additives selected from the group consisting of a metal stearate, stearic acid, a sucrose fatty acid ester, talc, and silicic acid are compression-molded.

11. The method for producing a compression-molded preparation according to claim 10, wherein the additive is magnesium stearate, calcium stearate, a sucrose fatty acid ester, or talc.

12. The method for producing a compression-molded preparation according to claim 10 or 11, wherein the drug is a drug having an unpleasant taste.

13. The method for producing a compression-molded preparation according to claim 12, wherein the drug having an unpleasant taste is one kind or two or more kinds selected from the group consisting of loxoprofen sodium, ibuprofen sodium, diclofenac potassium, diclofenac sodium, and naproxen sodium, or hydrates thereof.

14. The method for producing a compression-molded preparation according to any one of claims 10 to 13, wherein the polymer is a methacrylic polymer.

15. The method for producing a compression-molded preparation according to claim 14, wherein the methacrylic polymer is one kind or two or more kinds selected from the group consisting of a methyl methacrylate-diethylaminoethyl methacrylate copolymer, an ethyl acrylate-methyl methacrylate copolymer, a methacrylic acid-ethyl acrylate copolymer, and a methyl acrylate-methyl methacrylate-methacrylic acid copolymer.

16. The method for producing a compression-molded preparation according to any one of claims 10 to 15, wherein the coating of the polymer-coated, granulated substance with the additive is performed by mixing only the polymer-coated, granulated substance with the additive.

17. The method for producing a compression-molded preparation according to any one of claims 10 to 16, wherein the granules are coated with the additive in an amount of 0.01 to 5 parts by mass with respect to 100 parts by mass of the polymer-coated, granulated substance.

18. The method for producing a compression-molded preparation according to any one of claims 10 to 17, wherein the compression-molded preparation is an orally disintegrating tablet.

19. A granule, which is obtained by coating a polymer-coated, granulated substance, in which a granulated substance containing a drug is coated with a polymer coating film, with one kind or two or more kinds of additives selected from the group consisting of a metal stearate, stearic acid, a sucrose fatty acid ester, talc, and silicic acid.

20. The granule according to claim 19, wherein the additive is magnesium stearate, calcium stearate, a sucrose fatty acid ester, or talc.

21. The granule according to claim 18 or 19, wherein the drug is a drug having an unpleasant taste.

22. The granule according to claim 21, wherein the drug having an unpleasant taste is one kind or two or more kinds selected from the group consisting of loxoprofen sodium, ibuprofen sodium, diclofenac potassium, diclofenac sodium, and naproxen sodium, or hydrates thereof.

23. The granule according to any one of claims 19 to 22, wherein the polymer is a methacrylic polymer.

24. The granule according to claim 23, wherein the methacrylic polymer is one kind or two or more kinds selected from the group consisting of a methyl methacrylate-diethylaminoethyl methacrylate copolymer, an ethyl acrylate-methyl methacrylate copolymer, a methacrylic acid-ethyl acrylate copolymer, and a methyl acrylate-methyl methacrylate-methacrylic acid copolymer.

25. The granule according to any one of claims 19 to 24, wherein the content of the polymer is from 0.5 to 2000 parts by mass with respect to 100 parts by mass of the drug.

26. The granule according to any one of claims 19 to 25, wherein the content of the additive for coating the polymer-coated, granulated substance is from 0.01 to 5 parts by mass.

27. The granule according to any one of claims 19 to 26, wherein the granule is for use in a compression-molded preparation.

28. The granule according to any one of claims 19 to 27, wherein the granule is for use in an orally disintegrating tablet.
